Europäisches Patentamt

(19) European Patent Office

Office européen des brevets

(11) EP 0 787 794 A1

(12) EUROPEAN PATENT APPLICATION

(43) Date of publication:
06.08.1997 Bulletin 1997/32

(21) Application number: 97200275.2

(22) Date of filing: 30.01.1997

(51) Int. Cl.$^6$: C12N 1/16
// C12C11/00, A23J1/18

(84) Designated Contracting States:
BE CH DE DK ES FI FR GB IE IT LI NL SE

(30) Priority: 09.02.1996 IT MI960251

(71) Applicant: Asclepio Communications S.A.
Borgomaggiore (SM)

(72) Inventors:
• Puglisi, Pier Paolo
43100 Parma (IT)
• Ferrero, Iliana
43100 Parma (IT)

(74) Representative: Ferreccio, Rinaldo et al
Jacobacci & Perani S.p.A.,
Via Visconti di Modrone 7
I-20122 Milano (IT)

(54) Process for the aerobic adaptation of anaerobic yeasts

(57) A process is described for the aerobic adaptation of anaerobic yeasts, in particular brewers' yeast.

The process involves the suspension of a biomass of brewers' yeast obtained from beer fermentation tanks in a culture medium inside a fermenter, at a temperature of 20-32°C and a pH of 2.5-5.0, with sterile air being blown in at a rate equal to about 0.5-1.0 VVM, until a respiratory activity equivalent to a consumption of oxygen greater than or equal to 30 $\mu$l $O_2$/h/mg of yeast cells (dry weight) is achieved.

The invention also relates to the use of an aerobically adapted yeast as a dietary supplement in human food or as a component in feeds for the livestock industry.

EP 0 787 794 A1

**Description**

In its most general aspect, the present invention relates to a process for the aerobic adaptation of anaerobic yeasts.

In particular, the invention relates to a process for aerobically adapting brewers' yeasts used in anaerobic alcohol fermentation.

Brewers' yeast consists of yeasts of the genus Saccharomyces and is used to make beer by alcoholic fermentation of carbon sources such as, for example, cereal starches, in particular barley starch.

The beermaking process includes the formation of large amounts of a biomass consisting of yeasts which, at the end of the process, are separated out by sedimentation.

After drying, this biomass is used as a feed supplement in the zootechnical industry or as a dietary supplement in human food, on account of its content of proteins, mineral salts and vitamins, especially the group B vitamins.

During the beermaking process yeast follows an anaerobic metabolism and is consequently deficient in enzymatic co-factors and in catalytic activities specific to aerobic metabolism and, in general, it is deficient in reduced metabolites bearing active groups in the reduced state, such as, for example, glutathione and S-adenosylmethionine.

The enzymes at the end stages of oxidative metabolism are also absent, with a concomitant accumulation of organic acids of the tricarboxylic acid cycle in the mitochondrion, most notably in the adaptive metabolism and in the synthesis of those components of the mitochondrial membrane which, by means of the Mitchell proton gradient, contribute towards the intramitochondrial synthesis of ATP and towards reducing NAD and NADP to NADH and NADPH.

The enzymes involved in the synthesis of DNA and RNA and precursors thereof, which are characteristic of and specific to the mitochondrial compartment, are also absent.

The abovementioned deficiencies are essentially due to the missing functionality of the mitochondrial structures in brewers' yeast.

These deficiencies lead to an overall nutritional value for the brewers' yeast which is appreciably lower than that exhibited by aerobic yeasts such as, for example, bakers' yeast which, having fully functioning mitochondrial structures, is particularly rich in the abovementioned co-factors, amino acids and enzymes, as confirmed by the 50-80% increase in the doubling time of anaerobic yeast compared with its rate of doubling under aerobic conditions.

On the other hand, bakers' yeast is about 10-20 times more costly than brewers' yeast, which makes the use of bakers' yeast in sectors such as livestock feeds unfeasible.

The problem underlying the present invention is to make available a brewers' yeast which has a nutritional value similar to that of bakers' yeast, at appreciably lower cost.

This problem is solved, according to the invention, by a process for the aerobic adaptation of brewers' yeast, which comprises stages of:

i) removing a suspension of brewers' yeast containing from 10 to 15% solids from the bottom of beer fermentation tanks, at the end of the said fermentation,

ii) leaving the said suspension to settle, thereby producing a first sediment and a first supernatant, which is removed,

iii) resuspending the said first sediment in water and leaving it to settle, thereby producing a second sediment and a second supernatant, which is removed,

iv) resuspending the said second sediment in a suitable liquid culture medium and maintaining it at a temperature of 20-32°C and at a pH of 2.5-5.0, with sterile air being blown in at a rate equal to about 0.5-1.0 VVM, for a period which is sufficient to reach a respiratory activity in the brewers' yeast corresponding to a consumption of oxygen greater than or equal to 30 $\mu$l $O_2$/h/mg of yeast cells (dry weight),

v) recovering the yeast by centrifugation of the final suspension.

The time required to achieve the abovementioned respiratory activity in the brewers' yeast generally ranges from 15 to 40 hours.

The culture media used are preferably those chosen from the group comprising:

a) aqueous solutions containing 0.3-1.5% w/v yeast extract
b) aqueous solutions containing 0.3-1.5% w/v yeast extract and 0.1-1.5% peptone,
c) mineral minimum medium.

A mineral minimum medium which is particularly suitable for the purposes of the present invention is the mineral minimum medium/40 of Magni and Von Borstel (Genetics, Vol. 47, p. 1097, 1962).

According to a preferred embodiment of the invention, in the abovementioned stage iv), glucose is successively added to the said culture medium so as to keep its concentration constant at a value of 0.5-1% w/v.

The temperature at which stage iv) of the process is carried out is preferably between 24 and 29°C.

The appearance of respiratory activity in the brewers' yeast is detected by taking, at regular intervals, samples of the biomass of yeast which has undergone stage iv) of the process and evaluating the following two parameters for each sample:

a) the respiratory activity, expressed in $\mu$l of $O_2$ consumed per hour per mg of cells (dry weight) ($QO_2$);
b) the appearance of the absorption bands for cytochromes c, b and $aa_3$ within the range 540-620 nm.

The respiratory rate is measured using a Rank Brothers membrane oximeter, according to the following method.

A sample of the biomass is centrifuged at 1500 × g for 10 minutes, initially at 4°C, after which the supernatant is removed and the sediment is resuspended in distilled water at 4°C so as to obtain a concentration of $5 \times 10^9$ cells/ml.

50 microlitres of the abovementioned suspension are introduced into a cell in the oximeter, thermostatically set at 30°C, together with 2.9 ml of 0.1 M phthalate buffer at pH 5 and 5 microlitres of a glucose solution at a concentration of between 100 and 300 mM, as respiratory substrate.

The hourly consumption of $O_2$ per mg of dry weight is measured by introducing into the thermostatically-controlled cell the appropriate oximeter sensor, which is basically a Clark membrane electrode.

The appearance of the absorption bands for cytochromes c, b and $aa_3$ is detected using a Cary 219 double-beam spectrophotometer within the wavelength range from 540 to 620 nm, using as standard a solution of chromophores having an absorption of the same order of magnitude as the cell suspension but lacking absorption peaks within the abovementioned range.

The method used for the abovementioned detection involves centrifugation of the sample taken at room temperature (10-20°C) at 1500 × g for 10 minutes initially, followed by removal of the supernatant and resuspension of the sediment in water so as to obtain a cell concentration ranging between $1 \times 10^9$ and $5 \times 10^9$ cells/ml.

To determine the presence of cytochromes, scanning is carried out between 620 and 540 nm to locate the absorption peaks of cytochromes $aa_3$, b and c, which appear at 550, 560 and 602 nm respectively.

To measure the $aa_3$ peak, the baseline is plotted between 620 and 590 nm; to measure the b and c peaks, the abovementioned line is plotted between 570 and 540 nm.

The heights of the peaks thus located give an indication of the kinetics of the process of aerobic readaptation and of the acquisition of its structural bases (cytochromes c, b and $aa_3$) according to the formula:

$$mmol/g \ of \ cells = \frac{k \ x \ AU}{extinction \ coefficient \times g \ dry \ weight}$$

in which AU is the absorption determined from the height of the peaks and k is a coefficient which is a function of the range used.

Detection of the appearance of the abovementioned absorption bands is used as a means of screening in order to evaluate which strains of brewers' yeast are most suitable for aerobic adaptation and in order to define the structural sequence of the appearance of the lines for the respiratory cytochromes.

Confirmation that aerobic adaptation has taken place must, however, be furnished by detection of the "oxygen consumption" parameter, which directly and uniquely evaluates whether or not respiratory adaptation has taken place.

The present invention also relates to an aerobically adapted brewers' yeast, characterized in that its respiratory rate is greater than or equal to 30 $\mu$l $O_2$/h/mg of dry weight of yeast cells.

Such a yeast may be used as a dietary supplement in human food or as a component in feeds for the livestock industry.

Compared with the brewers' yeast currently used as a dietary supplement or as a component in feeds for the livestock industry, the brewers' yeast according to the invention has considerable advantages from the nutritional point of view.

Firstly, it is very rich in enzymic co-factors such as NADH and NADPH, and in sulphur-containing amino acids in reduced form (for example cysteine), as well as glutathione in reduced form. This is of prime importance in the prevention of degenerative phenomena and pathologies caused by oxidative processes involving free radicals.

Moreover, the brewers' yeast according to the invention is particularly rich in enzymes specific to the end stages of oxidative metabolism, which promote and increase intestinal assimilation of carbon sources produced by probiotic flora and/or which are present in feed formulations.

The process according to the present invention will be further described by means of the examples of implementation given purely by way of non-limiting illustration below.

EXAMPLE 1

A suspension of L 48 brewers' yeast from Interbreu Italia, with a solids content of 10%, was taken from the bottom of a tank for fermenting must for the production of beer, at the end of the abovementioned fermentation.

The suspension was left to settle and the supernatant was removed.

The sediment was resuspended in an equal volume of water. The suspension thus obtained was left to resettle and the supernatant was removed.

The sediment was then resuspended in an equal volume of a culture medium consisting of an aqueous solution containing 1% yeast extract. 8 litres of the suspension thus obtained were introduced into a 12-litre Chemap fermenter.

The temperature inside the fermenter was adjusted to about 25°C. The pH of the suspension was maintained at a value of about 4.5 using an automatic control system based on the use of pH sensors coupled to a pump system for delivering sodium hydroxide or hydrochloric acid solutions as and when required.

Sterile air was blown into the fermenter at an aeration rate equal to 1 VVM (volume of air per volume of suspension per minute) throughout the residence time of the suspension in the fermenter.

Samples of the biomass in suspension in the fermenter were taken at 4-hourly intervals and for each sample spectra of the respiratory cytochromes were acquired according to the method set forth above.

The absorbances corresponding to cytochromes $aa_3$, b and c were already present after 12 hours and reached a maximum after 24 hours.

Measurement of the respiratory activity, carried out 24 hours after the start of the process according to the method given above, confirmed that aerobic adaptation had taken place, giving an oxygen consumption value equal to 38 $\mu$l $O_2$/h/mg of yeast cells (dry weight).

Treatment of the yeast in the fermenter was therefore interrupted by stopping the insufflation of air, and the biomass of aerobically adapted brewers' yeast was removed from the fermenter and centrifuged at 1500 $\times$ g for 15 minutes at 4°C.

The yield for the process was equal to 100%.


EXAMPLE 2

A suspension of L 68 brewers' yeast from the company Poretti, with a solids content of 15%, was taken from the bottom of a tank for fermenting must for the production of beer, at the end of the abovementioned fermentation.

The suspension was left to settle and the supernatant was removed.

The sediment was resuspended in an equal volume of water. The suspension thus obtained was left to resettle and the supernatant was removed.

The sediment was then resuspended in an equal volume of a culture medium consisting of a mineral minimum medium according to Magni and Von Borstel.

8 litres of the suspension thus obtained were introduced into a fermenter identical to that used in Example 1.

The temperature inside the fermenter was adjusted to about 29°C. The pH of the suspension was maintained at a value of about 4.

Sterile air was blown into the fermenter at an aeration rate equal to 0.8 VVM (volume of air per volume of suspension per minute) throughout the residence time of the suspension in the fermenter.

Samples of the biomass in suspension in the fermenter were taken at 4-hourly intervals and for each sample spectra of the respiratory cytochromes were acquired according to the method set forth above.

Glucose solutions were successively added to the suspension so as to maintain its concentration at values of between 0.5 and 1% throughout the process.

The absorbances corresponding to cytochromes $aa_3$, b and c were already present after 12 hours and reached a maximum after 36 hours.

Measurement of the respiratory activity, carried out 24 hours after the start of the process according to the method given above, confirmed that aerobic adaptation had taken place, giving an oxygen consumption value equal to 35 $\mu$l $O_2$/h/mg of yeast cells (dry weight).

Treatment of the yeast in the fermenter was therefore interrupted by stopping the insufflation of air, and the biomass of aerobically adapted brewers' yeast was removed from the fermenter and centrifuged at 1500 $\times$ g for 15 minutes at 4°C.

The yield for the process was equal to 100%.

**Claims**

1. Process for the aerobic adaptation of brewers' yeast, which comprises stages of:

    i) removing a suspension of brewers' yeast containing from 10 to 15% solids from the bottom of beer fermentation tanks, at the end of the said fermentation,
    ii) leaving said suspension to settle, thereby producing a first sediment and a first supernatant, which is removed,
    iii) resuspending said first sediment in water and leaving it to settle, thereby producing a second sediment and a second supernatant, which is removed,

iv) resuspending said second sediment in a suitable liquid culture medium and maintaining it at a temperature of 20-32°C and at a pH of 2.5-5.0, with sterile air being blown in at a rate equal to about 0.5-1.0 VVM, for a period which is sufficient to achieve respiratory activity in the brewers' yeast corresponding to a consumption of oxygen greater than or equal to 30 $\mu$l $O_2$/h/mg of yeast cells (dry weight),

v) recovering the yeast by centrifugation of the final suspension.

2. Process according to Claim 1, in which said time ranges from 15 to 40 hours.

3. Process according to Claim 1, in which said culture medium is chosen from the group comprising:

a) aqueous solutions containing 0.3-1.5% w/v yeast extract
b) aqueous solutions containing 0.3-1.5% w/v yeast extract and 0.1-1.5% peptone,
c) mineral minimum medium.

4. Process according to Claim 3, in which, in said stage iv), glucose is successively added to the said culture medium so as to keep the glucose concentration constant at a value of 0.5-1% w/v.

5. Brewers' yeast, characterized in that its respiratory activity corresponds to an oxygen consumption greater than or equal to 30 $\mu$l/h/mg of yeast cells (dry weight).

6. Brewers' yeast which has been aerobically adapted using the process of any one of claims 1 to 4.

7. Use of a brewers' yeast according to either of Claims 5 and 6 as a dietary supplement in human food.

8. Use of a brewers' yeast according to either of Claims 5 and 6 as a component in feeds for the livestock industry.

9. Feed for the livestock industry comprising a brewers' yeast according to either of Claims 5 and 6.

10. Dietary supplement composition for human food, characterized in that it comprises a brewers' yeast according to either of Claims 5 and 6.

European Patent Office **EUROPEAN SEARCH REPORT**

Application Number

EP 97 20 0275

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DD 202 892 A (B. SEEGER ET AL.) 21 December 1981<br>* the whole document *<br>--- | 1,2,5,6 | C12N1/16<br>//C12C11/00,<br>A23J1/18 |
| A | DATABASE FSTA<br>INTERNATIONAL FOOD INFORMATION SERVICE (IFIS), FRANFURT/MAIN, DE<br>00169675, 1978<br>XP002031016<br>* abstract *<br>& TECHNOLOGIJA MESA,<br>vol. 19, no. 7/8, 1978, BELGRADE,YU,<br>pages 194-196,<br>P. MODIC ET AL.: "Nutritional and technological properties of a protein preparation made from brewer's yeast"<br>--- | 1,7-10 | |
| A,D | GENETICS,<br>vol. 47, 1962, BETHESDA,MD,US,<br>pages 1097-1108, XP000197466<br>G.E. MAGNI AND R.C. VON BORSTEL:<br>"Different rates of spontaneous mutation during mitosis and meiosis in yeast"<br>*see materials and methods*<br>--- | 1-3 | |
| A | EP 0 395 556 A (P. COHAS ET AL) 31 October 1990<br>* the whole document *<br>--- | 1,8,9 | |
| A | PROCEEDINGS OF THE 18TH EUROPEAN BREWERY CONVENTION,<br>1981,<br>pages 285-291, XP000197474<br>J. AHVENAINEN AND V. MAEKINEN: "The effect of pitching yeast aeration on fermentation and beer flavour"<br>* the whole document *<br>---<br>-/-- | 1-6 | |

TECHNICAL FIELDS SEARCHED (Int.Cl.6)

C12N

The present search report has been drawn up for all claims

| Place of search | Date of completion of the search | Examiner |
|---|---|---|
| BERLIN | 16 May 1997 | Mateo Rosell, A.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 0 787 794 A1

<table>
<tr><td colspan="2">European Patent Office</td><td>EUROPEAN SEARCH REPORT</td><td>Application Number<br>EP 97 20 0275</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US 1 722 746 A (J. HASLING JR.) 1 December 1924<br>* page 1, line 89-102 - page 2, line 1-12 *<br>--- | 1,6 | |
| A | DATABASE WPI<br>Week 8930<br>Derwent Publications Ltd., London, GB;<br>AN 89-219277<br>XP002031017<br>& SU 1 437 391 A (MAGARACH WINE PROD ) ,<br>15 November 1988<br>* abstract *<br>--- | 1-10 | |
| A | DATABASE WPI<br>Week 9130<br>Derwent Publications Ltd., London, GB;<br>AN 91-221548<br>XP002031018<br>& SU 1 592 329 A (FOOD BIOTECHN RESEARCH)<br>, 15 September 1990<br>* abstract *<br>----- | 1-10 | TECHNICAL FIELDS SEARCHED (Int.Cl.6) |

The present search report has been drawn up for all claims

| Place of search | Date of completion of the search | Examiner |
|---|---|---|
| BERLIN | 16 May 1997 | Mateo Rosell, A.M. |

EPO FORM 1503 03.82 (P04C01)